# EUROPEAN PATENT APPLICATION

(11) **EP 4 394 792 A1**
(43) Date of publication of application: **03.07.2024**
(21) Application number: 23218630.4
(22) Date of filing: 20.12.2023
(51) Int. Cl.: G16H 40/20, G06Q 10/06

(54) **DATASET GENERATION FOR HEALTHCARE FACILITY EVENTS**

(30) Priority: 31.12.2022 US 202218149091
(71) Applicant: TeleTracking Technologies, Inc., Pittsburgh, PA 15222 (US)
(72) Inventor: JUBECK, SCOTT, 15222 PITTSBURGH, PENNSYLVANIA (US); COEN, MICHAEL, 15222 PITTSBURGH, PENNSYLVANIA (US)
(74) Representative: Sandri, Sandro

(57) **Abstract**

One embodiment provides a method, the method including: identifying, using an event data identification and collection system, a plurality of events that can affect a healthcare facility; generating, using the event data identification and collection system and for each of the plurality of events, a set of questions identifying data that assists in supporting a given of the plurality of events; receiving, at the event data identification and collection system, an indication of an event affecting a healthcare facility; and providing, from the event data identification and collection system, the set of questions corresponding to the event affecting the healthcare facility. Other aspects are described and claimed.

## Description

### BACKGROUND

When an event (e.g., wildfire, cyberattack, tsunami, earthquake, hurricane, etc.) strikes, healthcare facilities may be uniquely affected. Since healthcare facilities provide healthcare and healthcare services to entire communities, regions, and/or the like, when an event strikes, more than just a single household is affected. Rather, healthcare facilities are critical to the infrastructure of a community and need to be supported and kept functioning as much as possible. In the event that a healthcare facility or portion thereof is no longer usable due to an event, the healthcare facility or portion thereof needs to be brought back online as quickly as possible. Accordingly, there are many mechanisms in place where other entities (e.g., federal governments, local governments, county governments, state governments, charities, associated healthcare facilities, etc.) may focus on healthcare facilities and assisting the healthcare facilities when an event occurs.

### BRIEF SUMMARY

In summary, one aspect provides a method including the steps described at claim 1. The dependent claims outline advantageous ways of carrying out the method.

Another aspect provides a system including: a processor; a memory device that stores instructions that, when executed by the processor, causes the system to carry out a method according to any one of the method claims.

A further aspect provides a computer program product including: a computer-readable storage device that stores executable code that, when executed by a processor, causes the product to: identify, using an event data identification and collection system, a plurality of events that can affect a healthcare facility; generate, using the event data identification and collection system and for each of the plurality of events, a set of questions identifying data that assists in supporting a given of the plurality of events; receive, at the event data identification and collection system, an indication of an event affecting a healthcare facility; and provide, from the event data identification and collection system, the set of questions corresponding to the event affecting the healthcare facility.

The foregoing is a summary and thus may contain simplifications, generalizations, and omissions of detail; consequently, those skilled in the art will appreciate that the summary is illustrative only and is not intended to be in any way limiting.

For a better understanding of the embodiments, together with other and further features and advantages thereof, reference is made to the following description, taken in conjunction with the accompanying drawings. The scope of the invention will be pointed out in the appended claims.

### BRIEF DESCRIPTION OF THE SEVERAL VIEWS OF THE DRAWINGS

FIG. 1 illustrates an example method of generating a set of questions for each of a plurality of events and providing an appropriate set of the questions when an event affects a healthcare facility.
FIG. 2 illustrates an example method of invoking a data transmission at a healthcare facility that is responsive to a request to provide data that assists in support the healthcare facility in view of an event affecting the healthcare facility.
FIG. 3 illustrates an example of device circuitry.

### DETAILED DESCRIPTION

When an event occurs that affects a healthcare facility, the healthcare facility may need many different resources, including staff, healthcare objects or supplies (e.g., wheelchairs, intravenous (IV) bags, medications, patient beds, procedure equipment, food, oxygen, etc.), objects to keep the healthcare facility functioning (e.g., generators, water purifiers, communication objects, etc.), and/or the like. Multiple entities exist which can assist in keeping healthcare facilities at least partly functioning when an event affects a healthcare facility. For example, governments at local through federal levels can provide supplies, staff, and/or other resources to assist the healthcare facility. As another example, charities may be able to provide resources and/or assist in taking care of patients who may have a need for a healthcare facility but that can be cared for at a lower level of care than a traditional healthcare facility, thereby reducing the strain on the healthcare facility. As another example, other healthcare facilities within a network of facilities may be able to take some of the patients of the affected facility, thereby reducing the burden of patients on the affected facility.

However, in order to assist the healthcare facility, the entities need to know what the healthcare facility needs. The needs can vary based upon the event that has occurred and that is affecting the healthcare facility. For example, if a hurricane has hit a healthcare facility, the healthcare facility may need emergency generators, communication devices that allow communication to other entities and/or healthcare facilities, additional patient beds, additional staff, and additional healthcare resources. As another example, if a wildfire is threatening a healthcare facility, the healthcare facility may need patient transfers or evacuations, additional staff, and/or the like. Additionally, while some types of resources may be common among different events, the specific resource may vary based upon the event. For example, for one event a particular type of healthcare object may see an increased need, whereas for a different type of event a different type of healthcare object may see an increased need. This is also true of healthcare staff, healthcare facility support departments (e.g., transport, janitorial, managerial, information technology, etc.), and/or other healthcare resources.

Conventionally, in order to determine the resources that a healthcare facility may need require the facility to manually provide the information to the requesting entity, for example, by calling, faxing, or otherwise communicating with the entity and providing a list of needs. Additionally, a healthcare facility may not know of a need for a resource until the resource has been depleted. In this case, the facility and entity must rush to fulfill the need. However, there is generally some commonality between needs and events, even if the events affect different healthcare facilities. For example, an disease outbreak may require additional staff regardless of the geographic location or healthcare facility that is affected by the disease outbreak. However, this commonality is not currently leveraged to anticipate needs by healthcare facilities in the case of an event.

Accordingly, the described system and method provides a system and method for generating a set of questions for each of a plurality of events and providing an appropriate set of the questions when an event affects a healthcare facility. An event data identification and collection system it utilized to identify a plurality of events that can affect a healthcare facility. The identified events are those that can affect any of a plurality of healthcare facilities. In other words, the events that are identified are not identified for a specific healthcare facility, a specific type of healthcare facility, a healthcare facility with specific requirements or resources, and/or the like. Thus, identified events may include any event that could affect any healthcare facility within a country, globally, and/or the like. Such events may include, but are not limited to, cyberattacks, hurricanes, earthquakes, wildfires, construction, flooding, blizzards, mudslides, nuclear attacks, radioactivity, tsunamis, terrorist attacks, power outages, and/or the like.

For each of the identified events, the event data identification and collection system (referred to herein as "system" for ease of readability), generates a set of questions identifying data that assists in supporting the event corresponding to the set of questions. For example, the system generates a set of questions that identifies data useful for supporting a wildfire affecting a healthcare facility. As another example, the system generates a set of questions that identifies data useful for supporting a hurricane affecting a healthcare facility. It should be noted that while the term "questions" is utilized, the information contained in the set of questions do not necessarily have to be traditional questions. Rather, the set of questions may identify questions to be asked, datapoints to be monitored, dashboards to be viewed and/or monitored, definitions to be provided, and/or any other information that may be useful to be queried, monitored, viewed, and/or otherwise provided. The system may also identify a frequency at which to review, monitor, request, and/or the like each information point within the set of questions.

When the system receives an indication of an event affecting a healthcare facility, the system may provide the set of questions that corresponds to the event to the healthcare facility and/or an entity attempting to assist the healthcare facility. Thus, the system provides a technique for generating a set of questions for a particular event that can be provided to a healthcare facility that is affected by an event or an entity that is attempting to support the healthcare facility so that resources of the healthcare facility can be monitored. This allows the healthcare facility and/or entity to quickly identify resources that will need to be supported in order to manage the effects of the event before the resources are unavailable, thereby allowing a more efficient process for supporting the healthcare facility and allowing the healthcare facility to focus on supporting the community needs as strongly as possible.

When an event affects a healthcare facility, one or more entities may assist in supporting the healthcare facility, for example, by providing resources, taking patients into other facilities, and/or the like. Some different entities that may assist in supporting a healthcare facility may include governments (e.g., local, county, state, federal, etc.), charities, other healthcare facilities, and/or the like. However, in order to effectively support the affected healthcare facility, the entity needs to know what resources are needed by the healthcare facility. As previously mentioned, the needed resources may vary based upon the event affecting the healthcare facility. Additionally, different healthcare facilities, different geographical regions, and/or the like, may dictate different needs for an affected healthcare facility. Accordingly, the affected healthcare facility needs to provide data to the one or more entities so that the one or more entities can effectively support the healthcare facility. The data may include an identification of resources or a status of resources that may be needed or utilized by the healthcare facility due to the event.

However, requesting the healthcare facility manually provide the necessary information to the entities requires the healthcare facility to take away resources that could be utilized for other purposes to monitor information and provide it to the entities. Conventionally, an entity can request information from the healthcare facility and the healthcare facility can provide the information via an electronic means. For example, the healthcare facility may start a communication transmission that includes the information requested by the entities. However, this conventionally requires a programming change effectuated by the entity responsible for the program or application that monitors the information and/or that provides the communication transmission. This means that there is a delay between the request for the information and provision of the information. Additionally, when the information is no longer requested, another programming change has to be made to remove the information from the communication transmission. These programming changes also result in the programming entity needing to allocate programming resources to change the program. Thus, this conventional technique for providing information or changing information provided to a requesting entity is inefficient and cumbersome.

Accordingly, the described system and method additionally provides a system and method for invoking a data transmission at a healthcare facility that is responsive to a request to provide data that assists in support the healthcare facility in view of an event affecting the healthcare facility. The healthcare facility employs the event data identification and collection system that may identify the healthcare facility is experiencing an event or that an event is affecting the healthcare facility. In view of this event, an entity that is attempting to support the healthcare facility may provide an indication to provide data that will assist in supporting the healthcare facility. The data may include resource levels, resource consumption, dashboards that provide different information, and/or the like. The data may be identified from the set of questions that correspond to the event and that were provided to the healthcare facility and/or supporting entity.

In response to this indication to provide data, the healthcare facility can create a modified dataset to be transmitted to the entity that includes the requested information. Creating the modified dataset may include modifying an original dataset that is already being provided to the entity to include the requested data. To create the modified dataset, the healthcare facility may identify a system that is already tracking the requested data and provide instructions to pull the data from the tracking system. In the case that the original dataset is a pointer to a data storage location, the healthcare facility may add the requested data to the data storage location. When a data transmission is invoked at the healthcare facility, the modified dataset will be accessed and transmitted to the requesting entity, thereby providing the requested data.

Thus, the system provides a technique that allows the healthcare facility to quickly change the information that is provided to a requesting entity without needing the programming entity to change the programming of the application or program. Additionally, the healthcare facility can set a time limit with the information transmission so that, upon expiration of the time limit, the newly provided information is no longer provided and the dataset of information provided reverts back to the original dataset. This results in a more efficient information transmission than conventional systems.

The described system and method will be discussed with respect to a healthcare system or healthcare facility for ease of readability. Additionally, for simplicity, the term "hospital" may be used here throughout. However, it should be understood by one skilled in the art that this term may refer to any healthcare system or facility, for example, long-term care facility, emergency department, healthcare staffing area, hospital facility including multiple departments within a healthcare campus or affiliations, affiliated or otherwise connected healthcare systems, and the like. However, the fact that this disclosure is discussed with respect to a healthcare system or hospital is not intended to limit this disclosure to only a healthcare system. As can be understood by one skilled in the art, the disclosed systems and methods can be applied to any entity that may need support from an outside entity.

The term "healthcare resource" will be generally used here throughout to refer to any resource that may be utilized within a healthcare facility or to support a healthcare facility. Thus, the term resource may encompass people, including healthcare professionals and support staff, healthcare objects used to treat or support treating patients (e.g., beds, procedure objects (e.g., scalpels, IV bags, forceps, stethoscope, etc.), wheelchairs, health monitoring devices, etc.), support departments (e.g., information technology, janitorial, housekeeping, managerial, transportation, etc.), healthcare facility support objects (e.g., communication devices, generators, water purifiers, etc.), and/or the like. This list is intended to merely be illustrative and not limiting.

Additionally, while the disclosure refers to an event affecting a healthcare facility, it should be noted that an event may only affect a portion of the healthcare facility or may affect the entire facility. For example, the operating room department may be closed, but the intensive care unit may remain open. As another example, the event may cause the entire facility to need to be shut down.

The illustrated example embodiments will be best understood by reference to the figures. The following description is intended only by way of example, and simply illustrates certain example embodiments.

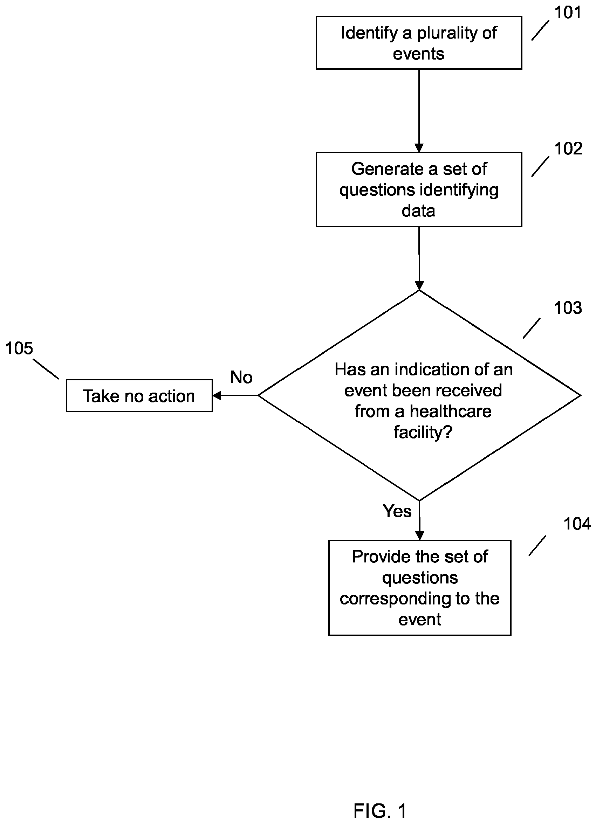

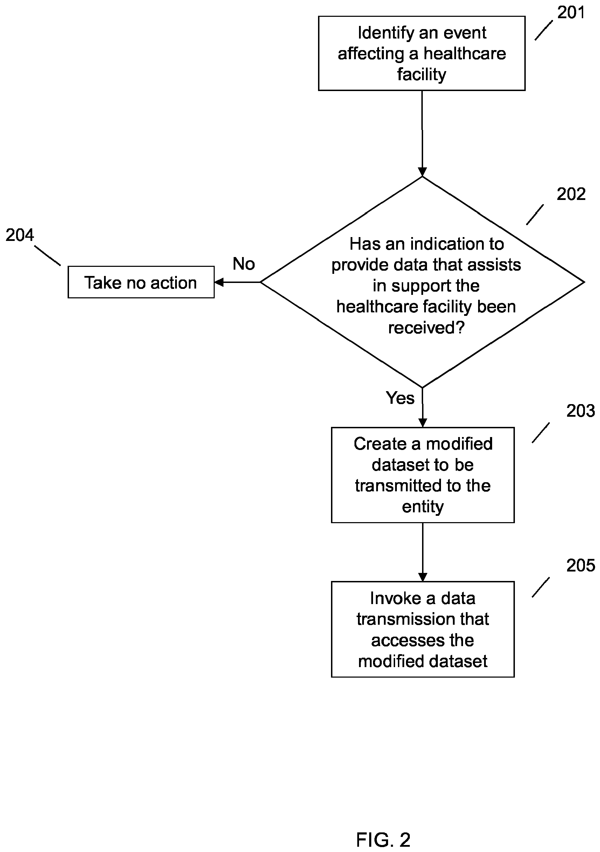

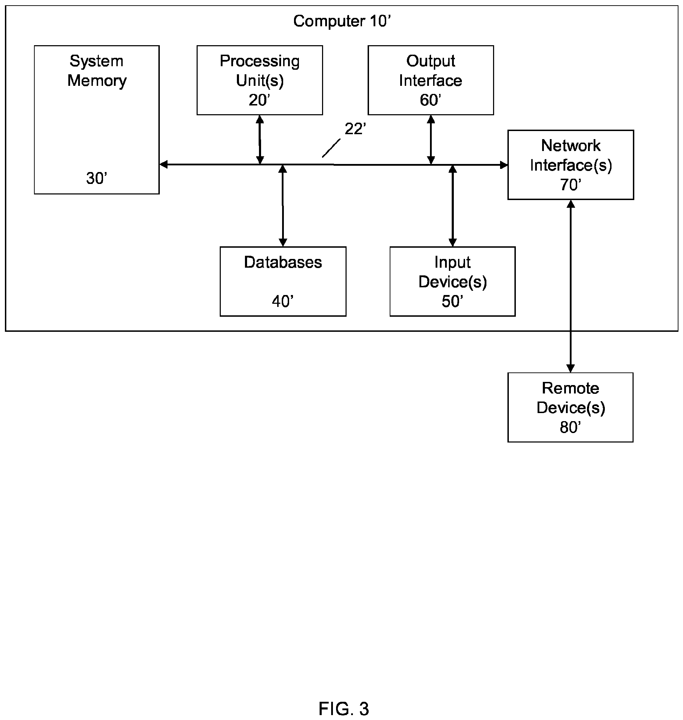

FIG. 1 illustrates a method for method of generating a set of questions for each of a plurality of events and providing an appropriate set of the questions when an event affects a healthcare facility. Additionally, FIG. 2 illustrates a method of invoking a data transmission at a healthcare facility that is responsive to a request to provide data that assists in support the healthcare facility in view of an event affecting the healthcare facility. The method of FIG. 1 and/or FIG. 2 may be implemented on a system which includes a processor, memory device, output devices (e.g., display device, printer, etc.), input devices (e.g., keyboard, touch screen, mouse, microphones, sensors, biometric scanners, etc.), image capture devices, and/or other components, for example, those discussed in connection with FIG. 3. While the system may include known hardware and software components and/or hardware and software components developed in the future, the system itself is specifically programmed to perform the functions as described herein to provide an event data identification and collection system for a healthcare facility that can generate a set of questions for each of a plurality of events that may affect a healthcare facility and that can invoke a data transmission that is responsive to a request to provide data that assists in supporting the affected healthcare facility. Additionally, the event data identification and collection system includes modules and features that are unique to the described system.

The event data identification and collection system, also referred to also as the system or event system, may include or have access to network devices and or communication components that facilitate communication between the event data identification and collection system and another device, component, facility, healthcare system, entity, and/or the like. For example, the event data identification and collection system may utilize a network device to communicate with the healthcare systems applications, programs, and/or systems, that are monitoring or tracking for events affecting the healthcare facility, monitoring or tracking healthcare facility resources, and/or the like.

The event data identification and collection system is provided that allows for interface between the healthcare facility and one or more entities that may be assisting in supporting the healthcare facility, particularly when the healthcare facility is affected by an event. It should be noted that while a single healthcare facility is discussed herein, the event data identification and collection system may be employed on many different healthcare facilities. Additionally, more than one healthcare facility may be affected by a single event, thereby having multiple healthcare facilities that need support due to a single event. Additionally, more than one event may affect a single facility or multiple events may occur that can affect multiple facilities across a geographical region. The event data identification and collection system may be utilized in any of these scenarios because it is employed on each healthcare facility and each instance of the event data identification and collection system does not interfere with other instances of the system. Additionally, where applicable, the event data identification and collection system employed on different healthcare facilities may be utilized to coordinate support between two or more of the facilities for one or more reasons.

The system may communicate and transmit information with other hospital systems that may provide information regarding patients, healthcare professionals, hospital objects, support personnel or departments, healthcare facility support resources, and/or other resources. For example, the system may interface with an object tracking system, patient registration system, employee system, support personnel or department system, emergency response systems, and/or the like. Thus, the system can pull information from each of the interconnected or accessible systems to identify information regarding resources of the healthcare facility. The system can also transmit information to one or more of the interconnected or accessible systems.

The system may be a background application that does not have an associated display or monitor. Rather, the system, when accessed by a user will provide instructions to generate and display a graphical user interface on a display device utilized by the user. Thus, the system may not have its own display device, but does have the ability to generate and display a graphical user interface. The graphical user interface is updated and managed based upon instructions provided by the system. In other words, the system generates and transmits instructions to create and update the graphical user interface.

Alternatively, or additionally, the system may be a standalone system that has an associated display device. In this case, the system still provides instructions for generating a graphical user interface as further described herein. The system may also be accessible through other computing devices. For example, the system may be a standalone system that can be accessed by a user and also may be an application that is accessible by a user on another computing device. The system may be accessible using any type of computing device, for example, personal computer, laptop computer, smartphone, tablet, smartwatch, head-mounted display, smart television or other smart appliance, and/or the like.

A graphical user interface that may be generated by the system may provide a user with the ability to provide an indication of an event affecting the healthcare facility. It should be noted that while a user or the healthcare facility may provide an indication of the event, other techniques for identifying an event and/or a healthcare facility affected by an event are contemplated and possible and are discussed in further detail herein. A graphical user interface may also provide a user with the ability to modify a dataset that is provided to an entity in order to provide data requested by the entity. Thus, the graphical user interface may include icons, fillable fields, pull-down menus, radial buttons, and/or a myriad of other graphical elements that allow for user interaction. Additionally, the graphical user interface may include graphical elements that provide information to the user. The layout of the graphical user interface may be a default configuration or configured by the user. It should be noted that different users may configure the graphical user interface per their preferences. Thus, the graphical user interface layout and configuration may be different between users. How much a user can configure the layout may be restricted or set by a system administrator and/or the like. Additionally, different users or different user roles may have different levels of access, which may also change how and what information is displayed.

Some icons or graphical elements may also be display icons that display information. The display icons may be static icons that have static visual elements. The display icons may be dynamic icons that have dynamic visual elements that change based upon an update in information displayed on the icon, periodically, based upon a condition being met, and/or the like. For example, the graphical element may be a display icon that displays the status of a provision of information to an entity, the status of a resource being utilized and monitored by the facility, the status of a resource being sent to the affected healthcare facility, and/or the like. In the example of a status dynamic icon, as the status is updated, the graphical element is updated to reflect the new status. In other words, the graphical element is iteratively updated based upon changes to the status of the schedule generation. As should be understood, there may be other dynamic graphical elements, and this is only an example of one. For example, other dynamic graphical elements may include other status indicators, statistic indicators, and/or the like.

At 101, the event data identification and collection system identifies a plurality of events that can affect a healthcare facility. An event that may affect a healthcare facility refers to an event that constrains or modifies the normal functioning of the healthcare facility. The possible events may be natural events, for example, earthquakes, tsunamis, wildfires, tornadoes, mudslides, floods, hurricanes, and/or the like. The possible events may be manmade events, for example, cyberattacks, terrorist attacks, construction, conflicts, and/or the like. Other events are also possible, for example, power outages, communication transmission outages, lack of potable water, food or other resource shortages, pandemics, disease transmissions, and/or the like.

To identify the possible events, the event data identification and collection system may utilize one or more identification techniques. The simplest technique may be by receiving user input identifying an event. One or more users may manually upload events to the system. The system may also utilize historical data to identify events that may affect a healthcare facility. In this case, the event data identification and collection system may monitor healthcare facilities, for example, those facilities where the system is deployed, and identify when the healthcare facility experienced or was affected to the point that the healthcare facility was no longer able to function normally.

Functioning normally may be a state that is learned by the system. For example, the system may monitor key datapoints, metrics, dashboards, and/or the like, to determine a normal value or range that those data indicate. Example data that may be monitored and may provide an indication of an abnormal functioning condition include staffing values, staff-to-patient ratios, patient bed occupancy, resource usage and availability ratios, and/or the like. Values for those data that run within the normal value or range may indicate a normal functioning of the healthcare facility. If the values meet or exceed a predetermined threshold, the system may identify that the facility is no longer functioning normally. To identify an abnormal functioning condition, the system may require that multiple data values are outside predetermined thresholds, that data values run outside the predetermined thresholds for a predetermined period of time, and/or the like. In addition to learning the normal functioning state values, a user may also program the system with some default values and threshold values.

Additionally, the system may monitor certain components or objects within or connected to the healthcare facility and may determine that an outage of one or more of those components or objects indicates an abnormal functioning state. For example, the system may monitor power to the healthcare facility, generator functioning, water availability, environmental water levels, weather conditions, and/or the like. The loss of one or more of these components may indicate an abnormal functioning state.

To identify a correlation between an abnormal operating state and an event, the system may identify an event that has occurred or is occurring proximal to the identification of the abnormal operating state. Thus, the system may identify a cause of the abnormal operating state and assign that cause as an event. To identify possible causes, the system may monitor one or more information sources that may provide an indication of an event. For example, news, social media sites, Internet searches, healthcare facility communications, and/or the like, may identify an event that has occurred, will be occurring, or is currently occurring that can be correlated to the abnormal operating state. For example, the system may identify that a news report indicated that a wildfire is burning in the area of the healthcare facility. Upon identifying an abnormal operating state, the system may correlate the wildfire as the event affecting the healthcare facility. A user may also manually identify the event causing the abnormal operating state. By identifying the cause of the abnormal operating state, the system has identified an event that can affect a healthcare facility.

Another technique for identifying an event may be to utilize crowd-sourced data or secondary sources. Many entities have manuals or other documentation that identifies the steps that should be taken when an event occurs at an entity. These events are those that affect the entity. In many cases, these identified events can also affect other entities. Accordingly, the system may access or be provided with entity documentation that includes an identification of events that can affect different entities in a type of crowd-sourcing potential event data. These events can then be identified as events that could affect a healthcare facility.

Part of identifying the events may be performed using one or more machine-learning models. A machine-learning model, which may be a neural network, decision tree and/or forest, classifiers, random tree forest or classifier, a combination thereof, a combination of machine-learning models, and/or the like, may be utilized in performing one or more acts of the described system. For example, one or more machine-learning models can be used to identify possible events, generate and/or modify sets of questions, identify that an event is affecting a healthcare facility, create a modified dataset, and/or the like. It should be understood that while the terminology may refer to a single machine-learning model, multiple machine-learning models can be utilized in performing one or more functions of the scheduling system. The machine-learning model may include a plurality of layers, including input, output, hidden, a combination thereof, and/or the like, layers. The machine-learning model is very complex and utilizes complicated mathematical computations. Due to the complexity of the machine-learning model, it would be impossible to perform the analysis as performed by the model in the human mind.

Additionally, the machine-learning model is trained to or utilized to make predictions on data that has been previously unseen by the model. To make these predictions, the model includes very complicated mathematical computations that would not be performed in the human mind. Rather, the use of a computer and processor, and, possibly a computer and processor that is specific and tuned to the machine-learning model, allows for performing these complex computations, particularly with a speed that allows for performing the complex processing found in and required by the machine-learning model in a time frame that facilitates the use of the machine-learning model for making the predictions. This speed is not possible with a human or even a group of humans. Thus, a human or even a group of humans, even using pen and paper, could not perform the analysis performed by the machine-learning model in a manner that would actually result in making the predictions provided by the machine-learning model on the large amount of data that is received by the system in a length of time that would make the system function as intended.

The machine-learning model may be trained using a training dataset having annotated training data. Annotated training data includes data that the model may make a prediction upon where the data is annotated with the correct prediction. The machine-learning model can learn from the training dataset how data should be classified or the predictions that should be made with respect to particular data. As predictions are made upon non-annotated data, feedback may be provided. Feedback may be provided in the form of a user making a correction, a user providing input regarding the prediction, predictions from other models regarding the same data, and/or the like. The feedback can be automatically ingested by the model to further train the model, thereby making the model more accurate over time. It should be noted that the model can monitor the predictions made by the model to identify the feedback so that a user does not have manually provide the feedback to the model. Thus, while the model may initially be trained with a training dataset, the model can continually be trained as it is deployed using predictions and feedback regarding the predictions. As an example, in the case of identifying events that could affect a healthcare facility, the machine-learning model may be trained using event data and may, therefore, be able to identify events that could affect a healthcare facility. Similar training and corresponding predications may be used for other portions of the described system. The machine-learning model also be trained using unsupervised learning techniques, other supervised learning techniques, reinforcement training, a combination thereof, and/or the like.

Other techniques for identifying events that could affect a healthcare facility are possible and contemplated. Additionally, it should be noted that multiple techniques, either those described or other techniques, can be used to identify possible events. Additionally, once events are identified a user may review the potential events and may choose to remove them as possible events for one reason or another.

At 102, the event data identification and collection system generates a set of questions for each of the events identified at 101. The set of questions identifies data that assists in supporting a healthcare facility that has been, is currently being, or will be affected by a given event. In other words, each set of questions identifies data from the healthcare facility that will be helpful in supporting the healthcare facility as it is affected by the event corresponding to the set of questions. As an example, the set of questions may be based upon identification of resources that are utilized for supporting a particular event. As an example, a power outage event may result in an increase in the use of generators for powering the healthcare facility. As another example, a flooding event may result in an increase in the need for potable water either transported in or created using a water purification system. As another example, a pandemic event may result in an increased need for healthcare staff. While each set of questions is unique to a particular event, it should be noted that many events may have common effects. Therefore, there may be the same or similar questions included in multiple sets of questions. Additionally, it is possible that some sets of questions are identical even if the sets of questions are identified as belonging to different events.

As previously noted, the sets of questions do not only include questions. Rather, the sets of questions may identify any data that may be useful to assist in supporting the healthcare facility. Thus, the sets of questions may include datapoints that should be monitored, dashboards that should be viewed, resources that should be monitored, and/or the like. The system may also identify a frequency at which the data identified in the set of questions should be viewed, monitored, updated, provided, accessed, and/or any other type of interaction. It should be noted that not all the data corresponding to the questions has to be tied to the same frequency of interaction. Rather, some data may correspond to one frequency of interaction, while other data may correspond to a different frequency of interaction.

As previously mentioned, generation of the set of questions may be performed utilizing a machine-learning model. The system may monitor healthcare facilities as they are experiencing an event and identify those data that can be correlated to the event. For example, the system may identify particular resources that are utilized during a particular event and a frequency of use of the resources, the system may identify a particular dashboard that is frequently accessed or that provides an indication of the operating state of the healthcare facility, the system may identify an outside source that indicates an attribute of the event which then affects an operation of the healthcare facility, and/or the like.

Similarly, the system may access historical information regarding events and the impact on healthcare facilities. From this historical information, the system can identify those data that should be subject to some interaction to support the healthcare facility by analyzing the information associated with the historical event. In this case, the system may analyze information for a historical event that has the same type as the event corresponding to the set of questions. In other words, if the set of questions corresponds to a particular event type, the system will analyze historical information corresponding to events of that same type. Other techniques for generating the questions are possible and contemplated, for example, a user may manually identify data that should be subject to some interaction, the system can monitor a healthcare facility while the healthcare facility is affected by a particular event type, the system may utilize a combination of techniques, and/or the like.

By identifying data that can be correlated to the event, the system can generate a set of questions that identifies those data that are correlated to the event. As previously noted, this data may include identification of dashboards, datapoints, information, resources, values, and/or the like, that should be monitored, obtained, accessed, and/or otherwise interacted with, that will assist a supporting entity in supporting the affected healthcare facility. In other words, the system identifies those data that are helpful in supporting the affected healthcare facility and then adds that data to the set of questions in whatever format the data is provided in (e.g., dashboard, datapoint, resource, etc.). Thus, the set of questions identifies those data that are useful for interaction in order to accurately and efficiently support the healthcare facility. For example, if the system identifies that a particular resource is utilized more frequently during an event, the system may include a monitoring of this resource within the set of questions. As another example, if the system identifies that a particular metric provides an indication of the operating state of the healthcare facility, the system may include a monitoring of this metric or dashboard within the set of questions.

Additionally, the set of questions for a given event may be updated or refined periodically based upon feedback. The feedback may be user feedback or system feedback. For example, the system may identify that a particular set of data is no longer applicable to the event for one reason or another. In this case, the system may remove any questions related to that data from the set of questions. Similarly, if the system identified that a set of data that has not been previously included within the set of questions is now applicable to the event, the system may update the set of questions to include questions regarding that set of data. Additionally, the system may test questions and determine the effectiveness of the question in determining how to support the healthcare facility while it is affected by an event. Based upon testing and/or feedback, the system can refine the questions included within the set of questions.

At 103, the event data identification and collection system may determine if an indication of an event affecting a healthcare facility has been received. The event data identification and collection system may receive an indication that an event is affecting a healthcare facility from the facility itself, from an entity attempting to support a healthcare facility, from a secondary source (e.g., news, social media site, healthcare facility communication transmissions, etc.), and/or the like. Identifying that an event is affecting a healthcare facility is discussed in more detail in connection with FIG. 2, step 201. Once an event affecting a healthcare facility has been identified, the event data identification and collection system may receive an indication of the event affecting a healthcare facility.

In addition to receiving an indication that an event is affecting a healthcare facility, the system may receive an indication of the type of event affecting the healthcare facility and attributes related to the healthcare facility that is being affected. Different healthcare facilities may have different characteristics or attributes that may require a modification of a set of questions. For example, a geographical location of a healthcare facility may dictate different resources, data, and/or the like, that may be useful as compared to other geographical locations. Thus, attributes of the healthcare facility may include a geographical location, resources of the healthcare facility, proximity to other entities, type of healthcare facility (e.g., long-term facility, emergency care facility, short-term recovery facility, general facility, etc.), connection to other healthcare facilities, and/or the like.

If, at 103, no indication of an event affecting a healthcare facility has been received, the system may take no action at 105. If, on the other hand, an indication of an event has been received at 103, the system may provide the set of questions corresponding to the event affecting the healthcare facility at 104. It should be noted that the set of questions may be provided to one or more entities, which may include the affected healthcare facility itself. The entities the set of questions is provided to may include those entities that are attempting to assist in supporting the healthcare facility.

In the event that an attribute of the healthcare facility dictates a change to the set of questions, the system may automatically make the modification before the set of questions is provided. To identify the modification that needs to be made, the system may utilize historical data, crowd-sourced data, machine-learning models, user input, and/or the like. These techniques have been previously described in connection with other steps and can be utilized in much the same except for modified to be for identifying modifications to the set of questions. Additionally, the system may receive an indication of a modification to the set of questions from the affected healthcare facility, which may occur before or after the set of questions is provided. This information can be used to make the modification to the set of questions and can also be stored for use in the future when a set of questions needs to be provided for the affected healthcare facility.

In response to the provision of the set of questions, an entity may then provide the set of questions to the healthcare facility so that the healthcare facility can be responsive to the set of the questions. This is discussed in more detail with respect to FIG. 2. In providing the set of questions to the healthcare facility, an entity may provide them in a format digestible by healthcare facility. When the set of questions are provided to the healthcare facility, either directly as the generated set of questions or indirectly from an entity, the event data identification and collection system may automatically pull data that is responsive to the set of questions from the healthcare facility. In other words, the set of questions can be pushed to the healthcare facility and connect to a tracking system, inventory system, scheduling system, and/or any other system of the healthcare facility that may provide responsive information, to pull information that is responsive to the set of questions. Instead of dynamically pulling the information from the healthcare facility systems, the healthcare facility may also dynamically change the information that is being published from the facility, as discussed in greater detail in connection with FIG. 2.

FIG. 2 illustrates an example method of invoking a data transmission at a healthcare facility that is responsive to a request to provide data that assists in support the healthcare facility in view of an event affecting the healthcare facility. At 201, an event affecting a healthcare facility is identified. In this step, not only is it identified that an event is affecting a healthcare facility, but it is also identified what type of event is affecting the healthcare facility. The healthcare facility employs an event data identification and collection system, which may be utilized to identify that an event is affecting the healthcare facility.

In identifying that an event is affecting a healthcare facility, the system may identify that the facility is operating within an abnormal operating state. Identifying the abnormal operating state can be performed utilizing a machine-learning model, historical information, and/or any other technique, for example, those described in connection with FIG. 1, step 101. In FIG. 1, identifying an abnormal operating state was utilized to identify different events that could affect a healthcare facility. In the case of step 201, identifying an abnormal operating state can be utilized to identify that a healthcare facility is currently being affected by an event. However, similar techniques can be used to identify that the healthcare facility is experiencing an abnormal operating state.

As another example for identifying an event, the system may employ a machine-learning model, as previously described, to identify an event is affecting a healthcare facility and to identify attributes about the event (e.g., event type, event duration, event geographic location, event range, etc.). As another example, the event data identification and collection system may monitor, mine, and analyze secondary sources (e.g., new sources, social media site, Internet searches, healthcare facility communications, etc.) which may be indicative of events affecting healthcare facilities. The system may be able to parse and mine the information to identify events and information related to the events. Depending on the medium of the secondary source, the system may employ one or more parsers (e.g., text parsers, audio parsers, image parsers, etc.), information extraction techniques (e.g., semantic analyzer, information extractors, parts-of-speech analyzers, syntactic analyzers, image analyzers, audio analyzers, etc.), machine-learning models, and/or the like, to analyze and extract information from the secondary sources.

Other techniques for identifying that an event is affecting a healthcare facility are contemplated and possible. For example, when an event affects a healthcare facility, a user within the facility may provide an indication that an event is affecting the healthcare facility. The user may also provide an indication of the type of event that is affecting the healthcare facility. One technique for providing this indication is to access a user interface which may include the graphical user interface discussed further herein. Within the graphical user interface the user may interact with one or more icons or graphical elements to indicate that an event is affecting the healthcare facility and may provide additional information regarding the event and/or affect on the healthcare facility. Another technique for a user providing this indication is a user could update a status of the healthcare facility on an Internet website or other secondary source. The system could then monitor this secondary source and identify that it has been updated to reflect that an event is affecting the healthcare facility.

In addition to identifying that an event is affecting the healthcare facility and identifying a type of the event affecting the healthcare facility, the system may also identify other characteristics regarding the event and/or healthcare facility. Example characteristics of the event other than the event type may include a range of the event, a current duration or expected duration of the event, a starting or expected starting time of the event, a severity of the event, and/or the like. Example characteristics of the healthcare facility include geographical location, proximity to other healthcare facilities, type of healthcare facility, capacity of the healthcare facility, unique characteristics of the facility (e.g., utilizes solar generators vs. fuel generators, has a water purification system vs. not having a water purification system, possible evacuation locations, etc.), and/or the like. The characteristics regarding the event and/or healthcare facility may assist in accurately and effectively supporting that specific healthcare facility during the event.

At 202, the event data identification and collection system within the affected healthcare facility determines if an indication to provide data that assists in supporting the healthcare facility in view of the event has been received. The indication to provide data may be received from an entity that will support the healthcare facility. The indication may be received through a user interface in the form of a request that identifies what information the supporting entity would like to know or needs in order to effectively support the affected healthcare facility. It should be noted that the healthcare facility may receive multiple requests for data due to the fact that multiple entities may be supporting the healthcare facility.

In addition to the indication to provide data, the indication may also include an identification of what data is requested by the supporting entity. The data that is identified may be based upon the set of questions that were generated by the event data identification and collection system for the event that is affecting the healthcare facility. Thus, when providing the indication or request for data, the supporting entity may also provide the set of questions that have been generated for the specific event that is affecting the healthcare facility. As previously mentioned, the requested data may be related to any datapoint, metric, dashboard, resource, and/or the like, that may assist in identifying how the supporting entity can support the healthcare facility. For example, the data may be related to a utilization of at least one resource of the healthcare facility. Thus, the question(s) related to this data may be to monitor a utilization level of the particular resource. As another example, the data may be related to an inventory of at least one resource of the healthcare facility. Thus, the question(s) related to this data may be to monitor an inventory level of the particular resource.

If an indication to provide data that assists in supporting the healthcare facility is not received at 202, the system may take no action at 204. Alternatively, the system may broadcast data that may be useful in supporting the healthcare facility to one or more entities. In other words, rather than reacting to a request for data that assists in supporting the healthcare facility, the system may proactively provide the data that assists in supporting the healthcare facility. In order to maintain privacy of protected information, the system may only broadcast data to entities for which a previous relationship has been established. For example, the system may only broadcast the data to entities which the healthcare facility is already in communication with. However, it is not strictly necessary that a previous relationship has been established and broadcasting may occur in a manner which is established by the healthcare facility and in accordance with the healthcare facility protocols or needs.

On the other hand, if an indication to provide data that assists in supporting the healthcare facility is received at 202, the system may create a modified dataset to be transmitted to the entity at 203. In creating the modified dataset, the system may modify an original dataset to include the data requested at 202. In some cases, the healthcare facility is already communicating with some entities that may become supporting entities. In this case, the healthcare facility may have a dataset that includes data that is already being communicated or transmitted to the entity. In this case, this dataset is the original dataset. In some cases, an original dataset may be an empty dataset, meaning that no dataset was previously created. For example, in the case of a charity or other entity that was not previously receiving information from the healthcare facility, the original dataset may be empty or non-existent. Thus, modifying an original dataset may also include the creation of a new dataset.

To identify the modified dataset, the system may utilize a predefined dataset that is responsive to the request. Alternatively, the dataset may be dynamically created as an event affects a healthcare facility. The healthcare facility may create or identify the data to be included in the modified dataset. Additionally, or alternatively, the system may utilize one or more techniques that automatically identifies the information to be included in the modified dataset that would be responsive to the requested data. For example, the system may utilize a machine-learning model, crowd-sourced techniques, historical information, and/or the like, to identify the information that should would be responsive to the request and, therefore, should be included in the modified dataset. This may include identifying the location of the data to be included in the modified dataset.

The original and/or modified dataset may include a group of data that is transmitted to the supporting entity. For example, the dataset, which will be used to refer to either the original dataset or modified dataset, may include files that include the data to be transmitted to the supporting entity. As another example, the dataset may include a pointer to a data storage location. Modifying the original dataset may then include adding the requested data to the data storage location. In this case, modifying the original dataset would not require that the pointer itself is modified since the data storage location does not change and instead only the data contained within the data storage location is changed.

As another example, at least some of the data that is requested by the supporting entity may already be tracked by another system of the healthcare facility, for example, a patient, staff, or object tracking system, an inventory system, a scheduling system, and/or the like. In this case, modifying the original dataset may include identifying the healthcare facility system that is already tracking the requested data and then providing instructions to pull the data from that healthcare facility system. The data may be pulled from the healthcare facility system and aggregated into a data storage location with other requested data which is then transmitted later or may be simply passed to the requesting entity directly from the healthcare facility system.

Other techniques for creating the modified dataset are contemplated and possible. For example, the event data identification and collection system may utilize a machine-learning model that can automatically modify the dataset based upon the request. In this case, the machine-learning model may parse the request and then determine the location of the requested data and then modify the dataset to include the requested data. As another example, a user may access a user interface, for example, the graphical user interface previously discussed, and provide user input at the user interface that creates the modified dataset, for example, by manually adding a pointer to the data, manually adding the requested data, and/or the like. As another example, the dataset may be stored in an Internet location and modifying the dataset may include modifying a series of webforms to include the requested data. Additionally, a combination of techniques for creating the modified dataset may be utilized, including those discussed herein or other creation techniques.

When modifying the original dataset, a length of time may be attached to the modification. The length of time may be a specific length of time or may a length of time that is tied to a trigger. For example, the requesting entity may specify how long the requested data is needed. Thus, the transmission of the modified dataset may be tied to this identified length of time. A user may also identify specific lengths of time, a default length of time may be identified, and/or the like. As another example, the system may monitor the healthcare facility and determine when the healthcare facility is operating at a normal operating state. Upon detecting the healthcare facility is operating at a normal operating state, the system may stop transmission of the modified dataset. Thus, a normal operating state may be a trigger. Other triggers are contemplated and possible, for example, when a resource reaches a predetermined inventory level, when a particular metric is met, when an indication of an event is over is received, and/or the like.

Other techniques for identifying a length of time are contemplated and possible. Once the length of time has been reached, the dataset may automatically revert from the modified dataset to the original dataset. In other words, once the length of time expires, the healthcare facility will no longer transmit the modified dataset and will instead transmit the original dataset, which may include no transmission at all.

At 205, the event data identification and collection system may, at the healthcare facility, invoke a data transmission that accesses the modified dataset and transmits the modified dataset to the requesting entity. The data transmission that is invoked may be based upon the how the dataset is stored and is to be transmitted. For example, in the case that a set of files including the modified dataset is utilized, the data transmission may be a file transfer application or transmission. As another example, in the case that the dataset includes a pointer to a data storage location, the data transmission may simply include transmitting the pointer. In this case, the pointer would not have to be modified because the data storage location remains the same. As another example, the dataset may be connected to an Internet location and invoking a data transmission may include invoking a series of webforms that transmit the modified dataset. Invoking a data transmission may include invoking an application programming interface (API) that allows for communication from the healthcare facility to the requesting entity.

The requested data will then be sent to the requesting entity until something occurs that causes the data to no longer be sent, for example, the expiration of the length of time, a user providing input to no longer transmit, a default setting, and/or the like. This allows the healthcare facility to have control over what data is sent to a requesting entity without needing to involve the programmer of the program to modify the programming in order to send a modified dataset.

It should be noted that the transmission of the modified dataset may include encryption in order to prevent an unauthorized entity from accessing the data. The encryption and subsequent decryption may be performed utilizing one or more encryption/decryption techniques, for example, asymmetric encryption, symmetric encryption, and/or the like. It should be noted that a combination of data transmission techniques may be utilized. For example, regardless of how the dataset is stored and is to be transmitted, the healthcare facility may invoke an application programming interface that allows for communication between the healthcare facility and the requesting entity.

While various other circuits, circuitry or components may be utilized in information handling devices, with a computer, server, client device or the like, an example device that may be used in implementing one or more embodiments includes a computing device in the form of a computer 10' as illustrated in FIG. 3. This example device may be a server used in one of the systems in a hospital network, or one of the remote computers connected to the hospital network. Components of computer 10' may include, but are not limited to, a processing unit 20', a system memory 30', and a system bus 22' that couples various system components including the system memory 30' to the processing unit 20'. Computer 10' may include or have access to a variety of computer readable media, including databases. The system memory 30' may include non-signal computer readable storage media, for example in the form of volatile and/or nonvolatile memory such as read only memory (ROM) and/or random access memory (RAM). By way of example, and not limitation, system memory 30' may also include an operating system, application programs, other program modules, and program data.

A user can interface with (for example, enter commands and information) the computer 10' through input devices 50'. A monitor or other type of device can also be connected to the system bus 22' via an interface, such as an output interface 360. The computer may include a database 40'. In addition to a monitor, computers may also include other peripheral output devices. The computer 10' may operate in a networked or distributed environment using logical connections to one or more other remote device(s) 80' such as other computers. The logical connections may include network interface(s) 70' to a network, such as a local area network (LAN), a wide area network (WAN), and/or a global computer network, but may also include other networks/buses.

Information handling device circuitry, as for example outlined in FIG. 2, may be used in client devices such as a personal desktop computer, a laptop computer, or smaller devices such as a tablet or a smart phone. In the latter cases, i.e., for a tablet computer and a smart phone, the circuitry outlined in FIG. 3 may be adapted to a system on chip type circuitry. The device, irrespective of the circuitry provided, may provide and receive data to/from another device, e.g., a server or system that coordinates with various other systems. As will be appreciated by one having ordinary skill in the art, other circuitry or additional circuitry from that outlined in the example of FIG. 3 may be employed in various electronic devices that are used in whole or in part to implement the systems, methods and products of the various embodiments described herein.

As will be appreciated by one skilled in the art, various aspects may be embodied as a system, method or device program product. Accordingly, aspects may take the form of an entirely hardware embodiment or an embodiment including software that may all generally be referred to herein as a "circuit," "module" or "system." Furthermore, aspects may take the form of a device program product embodied in one or more device readable medium(s) having device readable program code embodied therewith.

It should be noted that the various functions described herein may be implemented using instructions stored on a device readable storage medium, such as a non-signal storage device, and that are executed by a processor. A storage device may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random-access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a storage device is not a signal and "non-transitory" includes all media except signal media.

Program code embodied on a storage medium may be transmitted using any appropriate medium, including but not limited to wireless, wireline, optical fiber cable, RF, et cetera, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of connection or network, including a local area network (LAN) or a wide area network (WAN), or the connection may be made through other devices (for example, through the Internet using an Internet Service Provider), through wireless connections, e.g., near-field communication, or through a hard wire connection, such as over a USB connection.

Example embodiments are described herein with reference to the figures, which illustrate example methods, devices and program products according to various example embodiments. It will be understood that the actions and functionality may be implemented at least in part by program instructions. These program instructions may be provided to a processor of a device, a special purpose information handling device, or other programmable data processing device to produce a machine, such that the instructions, which execute via a processor of the device implement the functions/acts specified.

It is worth noting that while specific blocks are used in the figures, and a particular ordering of blocks has been illustrated, these are non-limiting examples. In certain contexts, two or more blocks may be combined, a block may be split into two or more blocks, or certain blocks may be re-ordered or re-organized as appropriate, as the explicit illustrated examples are used only for descriptive purposes and are not to be construed as limiting.

As used herein, the singular "a" and "an" may be construed as including the plural "one or more" unless clearly indicated otherwise.

## Claims

1. A method comprising:
identifying, using an event data identification and collection system, a plurality of events that can affect a healthcare facility;
generating, using the event data identification and collection system and for each of the plurality of events, a set of questions identifying data that assists in supporting a given of the plurality of events;
receiving, at the event data identification and collection system, an indication of an event affecting a healthcare facility; and
providing, from the event data identification and collection system, the set of questions corresponding to the event affecting the healthcare facility.

2. The method of claim 1, wherein the generating comprises accessing and analyzing historical data corresponding to at least one historical event having a same type as the event.

3. The method of claim 1, wherein the generating comprises utilizing a machine-learning model to generate the set of questions.

4. The method of claim 1, wherein the set of questions is based upon identifying resources utilized for supporting the given of the plurality of events.

5. The method of claim 1, wherein the generating comprises identifying at least one dashboard to be accessed during the given of the plurality of events.

6. The method of claim 1, wherein the generating comprises identifying at least one datapoint to be monitored during the given of the plurality of events.

7. The method of claim 1, wherein the generating comprises identifying a frequency for accessing the data.

8. The method of claim 1, wherein the generating comprises periodically refining the set of questions based upon feedback.

9. The method of claim 1, comprising receiving an indication of a modification to the set of questions corresponding to the event affecting the healthcare facility made by the healthcare facility.

10. The method of claim 1, comprising automatically pulling data, responsive to the set of questions, from the healthcare facility.

11. A system comprising:
a processor;
a memory device that stores instructions that, when executed by the processor, causes an information handling device to carry out a method according to any one of the preceding claims.

12. A computer program product comprising:
a computer-readable storage device that stores executable code that, when executed by a processor, causes the product to:
identify, using an event data identification and collection system, a plurality of events that can affect a healthcare facility;
generate, using the event data identification and collection system and for each of the plurality of events, a set of questions identifying data that assists in supporting a given of the plurality of events;
receive, at the event data identification and collection system, an indication of an event affecting a healthcare facility; and
provide, from the event data identification and collection system, the set of questions corresponding to the event affecting the healthcare facility.
